# EUROPÄISCHE PATENTSCHRIFT

(11) **EP 3 975 986 B1**
(45) Veröffentlichungstag und Bekanntmachung des Hinweises auf die Patenterteilung: **08.04.2026**
(21) Anmeldenummer: 20728083.5
(22) Anmeldetag: 29.05.2020
(51) Int. Cl.: A61K 9/00, A61K 9/20, A61K 31/485, A61K 9/28

(54) **ADDITIVES VERFAHREN ZUM DREIDIMENSIONALEN DRUCK WIRKSTOFFHALTIGER OBJEKTE**
ADDITIVE METHOD FOR 3D PRINTING ACTIVE INGREDIENT-CONTAINING OBJECTS
PROCÉDÉ ADDITIF D'IMPRESSION TRIDIMENSIONNELLE D'OBJETS CONTENANT DES PRINCIPES ACTIFS

(30) Priorität: 31.05.2019 EP 19177764
(43) Veröffentlichungstag der Anmeldung: 06.04.2022
(73) Patentinhaber: DiHeSys Digital Health Systems GmbH, 73525 Schwäbisch Gmünd (DE)
(72) Erfinder: DACHTLER, Markus, 89079 Ulm (DE); SCHLAUER, Alexander, 72622 Nürtingen (DE); HUBER, Gerald, 73655 Plüderhausen (DE)
(74) Vertreter: Habermann, Hruschka & Schnabel
(86) Internationale Anmeldenummer: PCT/EP2020/065096
(87) Internationale Veröffentlichungsnummer: WO 2020/240028

(56) Entgegenhaltungen:
- WO-A1-2017/158172
- WO-A1-2018/046642
- CN-A- 108 295 037
- NORMAN JAMES ET AL: "A new chapter in pharmaceutical manufacturing: 3D-printed drug products", ADVANCED DRUG DELIVERY REVIEWS, ELSEVIER, AMSTERDAM, NL, vol. 108, 18 March 2016 (2016-03-18), pages 39 - 50, XP029899913, ISSN: 0169-409X, DOI: 10.1016/J.ADDR.2016.03.001

## Beschreibung

Die vorliegende Erfindung betrifft ein additives Verfahren zur Herstellung fester oder halbfester dreidimensionaler Objekte, die einen oder mehrere pharmazeutische Wirkstoff(e) enthalten, sowie die durch das Verfahren hergestellten Objekte, wie halbfeste oder feste Darreichungsformen oder Medizinprodukte. Das Verfahren ist ein additives, vorzugsweise dreidimensionales Druckverfahren, bei dem einzelne definierte Volumeninkremente gedruckt werden, die hinsichtlich der enthaltenen Wirkstoffe, verwendeten Trägermaterialien, Form, Größe, Farbe, Wirkstoffkonzentrationen und Anordnung im hergestellten Objekt im Wesentlichen frei wählbar sind.

Aus der WO 2016/038356 A1 ist ein additives 3D-Druckverfahren zur Herstellung pharmazeutischer Darreichungsformen mittels Filament-Fusionsfabrikation (FFF) bekannt. Aus der WO 2017/158172 sind 3D-Druckverfahren für pharmazeutische Erzeugnisse bekannt, wobei die Voxel unterschiedliche Volumina aufweisen.

Aufgabe der vorliegenden Erfindung ist die Bereitstellung eines Verfahrens zur Herstellung wirkstoffhaltiger Objekte wie pharmazeutische Darreichungsformen oder wirkstoffhaltige Medizinprodukte, das eine erhöhte Flexibilität der Wahl unterschiedlicher Basismaterialien, Wirkstoffe und deren Verteilung in dem wirkstoffhaltigen Objekt ermöglicht.

Die vorstehende Aufgabe wird durch die Ausführungsformen der vorliegenden Erfindung, wie sie in den Ansprüchen, der vorliegenden Beschreibung sowie den beigefügten Figuren offenbart werden, gelöst.

Insbesondere stellt die vorliegende Erfindung ein Verfahren zur Herstellung eines dreidimensionalen Objekts bereit, das mindestens einen pharmazeutischen Wirkstoff enthält, umfassend die Schritte:
(i) Erstellen einer zwei- oder dreidimensionalen Widergabe des herzustellenden Objekts durch vordefinierte Volumeninkremente;
(ii) Drucken eines vordefinierten Volumeninkrements auf eine Aufbauvorrichtung oder auf ein auf der Aufbauvorrichtung angeordnetes Objekt;
(iii) Drucken eines weiteren Volumeninkrements derart, dass sich die Volumeninkremente mindestens teilweise berühren oder überlappen; und
(iv) Wiederholen der Schritte (ii) und (iii), bis das Objekt erstellt ist;
wobei mindestens eines der Volumeninkremente mindestens einen pharmazeutischen Wirkstoff enthält und die Volumeninkremente aus einer bei einer für den mindestens einen Wirkstoff verträglichen Drucktemperatur fließfähigen Basiszusammensetzung oder Basissubstanz umfassen, die sich nach dem Drucken des jeweiligen Volumeninkrements verfestigt und/oder die Volumeninkremente miteinander oberflächlich verklebt werden, dadurch gekennzeichnet, dass das Volumen der Volumeninkremente unterschiedlich ist derart, dass das Volumen der Volumeninkremente in dem gedruckten Objekt von außen nach innen ansteigt.

Der Ausdruck "dreidimensionales Objekt" ist erfindungsgemäß so zu verstehen, dass in der realen Welt sich jedes durch zwei- oder dreidimensionale Druckverfahren hergestellte Objekt, hier vor allem pharmazeutische Darreichungsformen und Medizinprodukte, in drei Raumrichtungen erstreckt. Sofern bei dem erfindungsgemäßen Verfahren auf der Aufbauvorrichtung oder auf ein auf der Aufbauvorrichtung bereits vorhandenes Objekt nur eine Schicht von sich mindestens teilweise berührenden Volumeninkrementen gedruckt wird, kann das erfindungsgemäße Verfahren auch als 2D-Druckverfahren beschrieben werden. Werden die Volumeninkremente bspw. als Tröpfchen aufgebracht, welche sich beispielsweise durch nachfolgenden Feuchtigkeitsentzug, z.B. durch Trocknung, makroskopisch als zweidimensional ausgedehnte Einheiten darstellen, so sind diese allerdings mikroskopisch dreidimensionale Strukturen, so dass erfindungsgemäß auch in solchen erfindungsgemäßen Ausführungsformen das Objekt eine dreidimensionale Ausdehnung aufweist.

Gemäß einer bevorzugten Ausführungsform des erfindungsgemäßen Verfahrens erfolgt der Aufbau des Objektes schichtweise, d.h. in den Schritten (ii) und (iii) werden die Volumeninkremente schichtweise gedruckt. Vorzugsweise werden dabei die Volumeninkremente zeilen- bzw. spaltenweise gedruckt.

Die vorliegende Erfindung kennzeichnet insbesondere die hohe Flexibilität der Zusammensetzung und der ermöglichten Vielzahl an Möglichkeiten des Aufbaus des hergestellten halbfesten oder festen Objekts. So können in bevorzugten Ausführungsformen des Verfahrens unterschiedliche Volumeninkremente unterschiedliche Wirkstoffe und/oder unterschiedliche Wirkstoffmengen und/oder unterschiedliche Basiszusammensetzungen bzw. Basissubstanzen aufweisen. Darüber hinaus kann die Form und/oder das Volumen der Volumeninkremente (im Folgenden auch "Voxels" genannt) gleich oder verschieden sein, wobei die Ansprüche verlangen, dass das Volumen der Volumeninkremente unterschiedlich ist derart, dass das Volumen der Volumeninkremente in dem gedruckten Objekt von außen nach innen ansteigt.

Erfindungsgemäß ist es jedoch auch vorgesehen, dass das Objekt vollständig aus Volumeninkrementen aufgebaut wird, die sämtlich einen einzigen, identischen Wirkstoff enthalten, wobei es erfindungsgemäß auch vorgesehen ist, dass jedes Volumeninkrement die gleiche Menge des Wirkstoffs bzw. die gleiche Konzentration des Wirkstoffs enthalten kann.

Die Volumeninkremente sind im Wesentlichen frei definierbar und können bspw. Tropfen, Kugeln, Punkte, Zylinder, Würfel, Quader oder sonstige Formen annehmen, wobei die genannten geometrischen Formen (Kugeln, Zylinder, Würfel, Quader) erfindungsgemäß so zu verstehen sind, dass die Voxels im Wesentlichen diese Gestalt, vorzugsweise, wenn sie nach dem Druck verfestigt sind, annehmen, so dass als erfindungsgemäß ebenfalls bevorzugte Voxelfomen auch allgemeiner Pellets, die bevorzugt einer Kugelform oder einem Zylinder angenähert sind, und Granulate zu nennen sind. Bevorzugte Voxelformen sind somit insbesondere tropfen-, pellet-, zylinder- und granutatförmige Voxels. Wie bereits vorstehend erläutert, können die Form (z.B. die vorstehend genannten Beispiele) und die Größe des Volumens der Volumeninkremente im Wesentlichen unabhängig voneinander frei kombiniert werden.

Ausführungsformen des erfindungsgemäßen Verfahrens bedienen sich der prinzipiell freien Wählbarkeit der Volumina der geruckten Volumeninkremente: Grundsätzlich schrumpft das Volumen einer pharmazeutischen Darreichungsform bei deren Abbau hin zum bzw. am Freisetzungsort von außen nach innen. Dies bewirkt, dass die Menge des pro Zeiteinheit freigesetzten Wirkstoffs abnimmt. Um eine in dieser Hinsicht möglichst gleichmäßige Wirkstofffreisetzung über den Verlauf des Abbaus der Darreichungsform zu erreichen, ist es erfindungsgemäß vorgesehen, die Volumeninkremente derart zu drucken, dass das Volumen der gedruckten Volumeninkremente von außen nach innen ansteigt. Dies wird erfindungsgemäß durch das Drucken entsprechender Schichten von Volumeninkrementen realisiert, wobei das Volumen der Volumeninkremente von Schicht zu Schicht oder von Gruppe von Schichten gleichen Volumens zu weiteren Schichten gleichen Volumens von außen nach innen ansteigt.

Wie bereits ausgeführt, können unterschiedliche pharmazeutische Wirkstoffe (sog. APIs, engl. active pharmaceutical ingredients) mit Hilfe des erfindungsgemäßen Verfahrens in dem Objekt enthalten sein. Darüber hinaus können in den Volumeninkrementen mehrere (also zwei oder mehr) Wirkstoffe enthalten sein. Es können selbstverständlich auch Volumeninkremente so gedruckt werden, dass jedes Volumeninkrement einen API enthält, jedoch unterschiedliche APIs (zwei oder mehr) in getrennten Volumeninkrementen vorliegen. Es können auch Volumeninkremente gedruckt werden, die unterschiedliche Konzentrationen (also Wirkstoffmenge pro Volumeninkrement) eines pharmazeutischen Wirkstoffs enthalten. Das Verfahren kann in einer diesbezüglichen Ausführungsform so gestaltet werden, das wirkstoffhaltige Volumeninkremente derart aufgebaut sind und gedruckt werden, dass mindestens eine erste Gruppe von sich berührenden oder überlappenden wirkstoffhaltigen Volumeninkrementen eine gleiche Wirkstoffmenge enthält und mindestens eine zweite Gruppen von sich berührenden Volumeninkrementen eine Wirkstoffmenge enthält, die von der Wirkstoffmenge der ersten Gruppe verschieden ist. So können in einem durch das Verfahren hergestellten Objekt Konzentrationsgradienten aufgebaut werden. Auch diese Ausführungsform der Erfindung wird in bevorzugten Varianten der Erfindung zur Bereitstellung einer gleichmäßigen Wirkstofffreisetzung verwendet, wie oben für die Erhöhung der Volumina der Volumeninkremente in der Darreichungsform von außen nach innen beschrieben. Dabei werden zur Bereitstellung einer möglichst gleichmäßigen Freisetzung des Wirkstoffs oder der Wirkstoffe die Volumeninkremente bevorzugt derart gedruckt, dass die Wirkstoffkonzentration vorzugsweise in den Volumeninkrementen von außen nach innen ansteigt.

Bei der Herstellung von Objekten gemäß dem erfindungsgemäßen Verfahren können Gruppen von Volumeninkrementen mit unterschiedlichen pharmazeutischen Wirkstoffen gebildet werden. Dabei kann mindestens eine erste Gruppe wirkstoffhaltiger Volumeninkremente vorhanden sein, die einen ersten pharmazeutischen Wirkstoff enthält und mindestens eine zweite Gruppe wirkstoffhaltiger Volumeninkremente vorhanden sein, die einen vom ersten Wirkstoff verschiedenen zweiten pharmazeutischen Wirkstoff enthält. Die unterschiedlichen Gruppen von Volumeninkrementen können dabei so gedruckt werden, dass sie innerhalb des Objekts zusammengefasst sind. Das heißt, dass die Volumeninkremente der ersten und/oder der zweiten Gruppe (sowie ggf. jeder weiteren Gruppe, falls mehr als zwei Wirkstoffe in dem zu druckenden Objekt vorhanden sein sollen) derart gedruckt werden, dass sich die Volumeninkremente der jeweiligen Gruppe berühren.

Es ist in weiteren Ausführungsformen der Erfindung auch vorgesehen, dass wirkstoffhaltige Volumeninkremente derart gedruckt werden, dass sie innerhalb des Objekts eine oder mehrere Gruppen bilden, die mindestens teilweise, in anderen Ausführungsformen auch vollständig durch nicht wirkstoffhaltige Volumeninkremente umgeben sind, welche die wirkstoffhaltigen Volumeninkremente von der äußeren Umgebung trennen bzw. abschirmen, so dass bspw. ein Objekt mit einem wirkstoffhaltigen Kern oder jedenfalls eine innere Gruppe von miteinander verbundenen wirkstoffhaltigen Volumeninkrementen (oder mehreren inneren Gruppen von benachbarten Volumeninkrementen mit gleichen oder unterschiedlichen Wirkstoffen bzw. gleichen oder unterschiedlichen Wirkstoffmengen) erstellt wird, um den herum nicht wirkstoffhaltige Volumeninkremente angeordnet sind. Die "äußere Umgebung" kann dabei das das Objekt umgebende Milieu sein. Unter "äußerer Umgebung" um einen Kernbereich oder eine innere Gruppe von einander unmittelbar verbundenen Volumeninkrementen wird vorliegend auch eine anderer Bereich innerhalb des gedruckten Objekts verstanden, d.h. nicht wirkstoffhaltige Volumeninkremente können im erfindungsgemäßen Objekt wirkstoffhaltige Gruppen von Volumeninkrementen mindestens teilweise, ggf. auch vollständig von anderen einzelnen oder Gruppen von Volumeninkrementen, die bspw. einen anderen (oder mehrerer andere) Wirkstoff(e) enthalten umgeben, um so Trennschichten oder Trennbereiche zwischen den unterschiedlich bestückten Volumeninkrementen zu bilden.

Derartige Anordnungen können in bevorzugten Ausführungsformen zur räumlichen Isolierung der einzelnen wirkstoffhaltigen Volumeninkremente genutzt werden, um z.B. chemische Instabilitäten der einzelnen Wirkstoffe zu vermeiden und/oder unterschiedliche Wirkstoffe, die chemisch nicht miteinander verträglich sind (da sie bspw. miteinander reagieren oder in sonstiger Weise ihre Struktur und/oder Wirksamkeit beeinträchtigen), voneinander zu trennen.

In anderen Ausführungsformen des vorgenannten Typs können auch bspw. Drug-Abuse-Deterrent-Tabletten oder -Kapseln bereitgestellt werden, die es verhindern, dass z.B. ein Wirkstoff (bspw. Opioide oder Wirkstoffe mit Suchtpotential) bspw. durch Zerkleinerung oder in anderer Weise aus einer Darreichungsform gewonnen und einer missbräuchlichen Verwendung zugeführt werden kann. So werden in bevorzugten Ausführungsformen der Erfindung Gruppen oder Schichten von Volumeninkrementen mit dem oder den Wirkstoff(en) (bspw. den vorgenannten potentiell missbräuchlich verwendbaren Substanzen) gedruckt, die von Gruppen oder Schichten von Volumeninkrementen umgeben sind, die eine Substanz enthalten, welche die Wirkung des (oder der) Wirkstoffs (Wirkstoffe) aufhebt, den oder die Wirkstoffe abbaut oder in anderer Weise die potentiell missbräuchliche Verwendung des oder der Wirkstoffe mindestens begrenzt, besonders bevorzugt verhindert. Es können zwischen den Gruppen (oder Schichten) von Wirkstoff(en) und Missbrauchsverhinderungssubstanz(en) auch eine oder mehrere Gruppen oder eine oder mehrere Schichten von Volumeninkrementen vorgesehen sein, welche keinen Wirkstoff und keinen den Missbrauch verhindernde Substanz enthalten (in bevorzugten Ausführungsformen werden diese Volumeninkremente lediglich die verwendete Basisaufbausubstanz enthalten) und die wirkstoffhaltigen Volumeninkremente von den Volumeninkrementen mit der/den Missbrauchsverhinderungssubstanzen trennen.

Weiterhin können solche Ausführungsformen mit wirkstoffhaltigen Volumeninkrementen, die zumindest teilweise von nicht-wirkstoffhaltigen Volumeninkrementen umgeben sind, erfindungsgemäß z.B. für die Herstellung von den oder die Wirkstoff(e) langsam freisetzenden Ausführungsformen wie Retard-Tabletten oder -Kapseln oder magensaftresistenten Tabletten oder Kapseln, genutzt werden.

Das Verfahren der vorliegenden Erfindung kann somit zur Herstellung von Objekten, insbesondere pharmazeutischen Darreichungsformen verwendet werden, welche den oder die API(s) an einem gewählten Ort oder einem gewählten Bereich der gewünschten Applikation freisetzen (sog. "Drug-Targeting"), d.h. vorzugsweise zur Freigabesteuerung des oder der Arzneistoffe aus der gedruckten Arzneiform verwendet werden. Derartige Ausführungsformen dienen also dazu, den Arzneistoff oder die Arzneistoffe an den optimalen Wirkort oder Zielort, beispielsweise (und bevorzugt) nach einer oralen Verabreichung, zu bringen. So ist es in bestimmten Ausführungsformen der Erfindung vorgesehen, dass die Volumeninkremente derart gedruckt werden, dass ein Kernbereich von Volumeninkrementen einer erfindungsgemäßen pharmazeutischen Darreichungsform einen oder mehrere gewünschte Wirkstoff(e) enthält und um diesen Kernbereich (oder um dieses Kernvolumen) sich ein oder mehrere Schichten von Volumeninkrementen angeordnet sind, die bspw. pH-abhängig im Darm abgebaut bzw. (auf-)gelöst werden, so dass der Kernbereich erst durch den pH-abhängigen Abbau der äußeren Schicht(en) in dem vorgewählten Bereich des Darms dem umgebenden Milieu ausgesetzt wird und dort den oder die Wirkstoff(e) freisetzt. Dies wird meist durch in der Aufbausubstanz vorhandene, im Fachgebiet wohlbekannte Polymere (wie beispielsweise Schellack, Copolymere von Methacrylsäure und Methacrylmethacrylat, modifizierte Cellulosen usw.) erreicht, deren pH-abhängiger Abbau bzw. pH-abhängige Löslichkeit sehr fein steuerbar ist, so dass eine pH-abhängige Freilegung des wirkstoffhaltigen Kernbereichs der Darreichungsform für jeden Abschnitt des Darms, insbesondere Dünndarms (Duodenum, Jejunum und Ileum) erfindungsgemäß bereitgestellt werden kann. Die Bereitstellung einer gezielten Freisetzung an einem bestimmten Wirkort oder Zielort, wie beispielsweise dem Darm oder einem gewählten Darmabschnitt, ist nicht auf pH-abhängig abbaubare bzw. pH-abhängig lösliche Schichten aus Volumeninkrementen mit entsprechenden pH-abhängig abbaubaren bzw. pH-abhängig löslichen Polymeren, die in derartigen Aufbausubstanzen enthalten sind, beschränkt. Es können auch alternativ oder zusätzlich andere Mechanismen realisiert werden. So können erfindungsgemäß die Volumeninkremente derart gedruckt werden, dass sich eine oder mehrere Schichten von Volumeninkrementen, beispielsweise direkt auf einem wirkstoffhaltigen Kernbereich oder auf einer oder mehreren ggf. vorhandenen Zwischenschicht(en) ergeben, deren Aufbausubstanz einen bakteriell abbaubaren Anteil enthält oder daraus besteht. Hierfür eignen sich z.B. einem Fachmann bekannte bakteriell abbaubare Polymere wie bpsw. Stärken oder Cellulosen. Derartige Schichten dienen vorzugsweise zur Freisetzung von Wirkstoffen im Kolon. In bevorzugten Ausführungsformen können die vorstehend genannten Schichten, z.B pH-abhängig abgebaute Schichten (ein oder mehrere) und bakteriell abbaubare Schichten kombiniert werden. Es können so auch bspw. Darreichungsformen für pharmazeutische Wirkstoffkombinationen gedruckt werden, bei dem in einem Kernbereich Volumeninkremente mit einem ersten Wirkstoff angeordnet werden, der von einer oder mehreren Schichten von Volumeninkrementen umgeben ist, die bakteriell abbaubare Substanzen in der Aufbausubstanz enthalten (oder daraus bestehen). Darauf folgt/folgen eine oder mehrere Schichten mit Volumeninkrementen, die einen zweiten Wirkstoff enthalten, wonach eine oder mehrere Schichten folgen, welche einen oder mehrere pH-abhängig abbaubare Polymer(e) in der Aufbausubstanz enthalten (oder daraus bestehen). Alternativ kann natürlich auch bei einer derartigen Ausführungsform mit mehreren Drug-Targetng-Schichten auch nur der Kernbereich ein oder mehrere Wirkstoffe enthalten.

Da das Verfahren auch unter Sterilbedingungen durchführbar ist, kann das erfindungsgemäße Druckverfahren auch zur Bereitstellung von Implantaten und/oder wirkstofffreisetzenden Injektionen oder Wirkstoffdepots angewandt werden.

Weiterhin trägt erfindungsgemäß auch zur erhöhten Flexibilität des Verfahrens bei, dass sehr unterschiedliche Materialien (Wirkstoffe und Basiszusammensetzungen bzw. -substanzen) für die zu druckenden Volumeninkremente verwendbar sind.

Die Bindung zwischen den einzelnen aufgebrachten Volumeninkrementen kann auf unterschiedliche Weise erfolgen. In einer Ausführungsform kann bspw. bei Verwendung eines schmelzbaren Materials die Bindung zwischen solchen Voxels durch die Verfestigung nach der Aufbringung auf die Trägerstruktur erfolgen, wobei diese durch verschiedene Mechanismen wie simple Abkühlung und/oder chemisch durch bekannte Substanzen durchgeführt werden kann. In einer anderen Ausführungsform kann dem Voxelmaterial z.B. einer Dispersion oder einer Lösung ein geeignetes Bindemittel zugesetzt werden, dass nach dem Aufbringen des Voxels dieses zum Aushärten bringt, wobei die Aushärtung durch das Bindemittel bspw. durch Wärme erfolgen kann, die durch eine geeignete Wärmequelle in der Druckvorrichtung zugeführt werden kann wie bspw. einer Lichtquelle, vorzugsweise einer Laser-Einrichtung. Das Aushärten durch das Bindemittel kann auch chemisch durch entsprechende Startermoleküle und/oder Licht einer geeigneten Wellenlänge erfolgen, wobei wiederum letzteres bevorzugt mit Hilfe einer Lasereinrichtung emittiert wird. In einer weiteren Ausführungsform kann das Fluid des Volumeninkrements ein oder mehrere Ausgangsverbindungen, typischerweise Monomere, eines oder mehrerer Polymere enthalten und nach dem Aufbringen des Voxels wird durch geeignete Mittel wie bspw. wiederum Licht, Wärme oder andere Polymerisationsstarter eine Polymerisation in Gang gesetzt, welche das aufgebrachte Voxel aushärtet und mit benachbarten Voxels verbindet bzw. verklebt.

Geeignete bei der Drucktemperatur fließfähige Träger-Materialien, in welchen der oder die pharmazeutischen Wirkstoff(e) vorliegt/vorliegen, sind z.B. im Allgemeinen für eine Schmelzextrusion (engl. hot melt extrusion, HME) verwendbare Träger wie niedrig schmelzende Wachse und Polymere. Das HME-Gemisch bzw. allgemein das Volumeninkrementgemisch kann neben dem niedrig schmelzenden Träger weitere Prozessmittel und Hilfsstoffe wie Bindemittel, Weichmacher, Antioxidantien, Duftstoffe, Süßstoffe oder ähnliches enthalten. Geeignete HME-Träger und Weichmacher und sind z.B. in Crowley et al. (2007) Drug Development and Industrial Pharmacy, 33,909-926, offenbart (Träger: Seiten 917 bis 919, insbesondere Tabelle 1; Weichmacher: Seiten 917 und 920, insbesondere Tabelle 2), wobei in der vorliegenden Beschreibung *expressis verbis* auf die genannten Passagen Bezug genommen wird.

Das erfindungsgemäße Verfahren ist nicht auf einen vollständigen *De-Novo*-Aufbau von Darreichungsformen oder Medizinprodukten beschränkt. Das Verfahren kann auch auf bereits auf der Aufbauvorrichtung vorhandene Objekte angewandt werden. Eingeschlossen hierin sind bspw. zuvor konventionell oder in anderer Weise hergestellte Darreichungsformen, die bspw. durch das vorliegende Verfahren modifiziert werden sollen, oder wirkstofffreie Objekte (auch Placebo-Träger genannt), auf die wirkstoffhaltige Volumeninkremente in erfindungsgemäßer Weise aufgedruckt werden. So können bspw. wirkstofffreie Folien oder andere flächige Materialien wie Esspapier vorgelegt werden, um z.B. wirkstoffhaltige ODF ("orally degradable film" oder "orally dissolvable film") -Produkte bereitzustellen. In anderen Ausführungsformen können vorgelegte Pflastermaterialien bpsw. mit Volumeninkrementen erfindungsgemäß bedruckt werden, die bspw. wundheilungsfördernde Wirkstoffe enthalten. In weiteren Ausführungsformen können Placebo-Träger, die bspw. durch ein Fused Layer Modeling-Verfahren hergestellt wurden, durch das erfindunggemäße Verfahren mit API-haltigen Volumeninkrementen und anschließendem Druck von Lösungsmittelhaltigen Flüssigkeiten bedruckt werden, wobei sich die gedruckten Schichten abwechseln können.

Das vorliegende additive 3D-Druckverfahren wird bevorzugt computergestützt durchgeführt. So wird typischerweise im Schritt (i) ein berechnetes dreidimensionales Bild des zu druckenden Objekts z.B. mit Hilfe eines gängigen CAD-Programms erstellt. Die computergenerierte Widergabe des zu druckenden Objekts kann auch durch scannen einer bereits existierenden Darreichungsform erfolgen. Das computergenerierte Modellbild wird dann bei dem vorliegenden Verfahren in die gewünschten, im Prinzip frei wählbaren Volumeninkremente (Voxel) unterteilt, wobei die Auflösung des realen Objekts umso größer wird, je kleiner die Volumeninkremente sind. Jedem einzelnen Volumeninkrement kann bspw. ein Wirkstoff, Träger- oder Basissubstanz bzw. Träger- oder Basiszusammensetzungen und/oder weitere Hilfsmittel wie Farbsubstanzen und andere ggf. benötigte Materialien sowie deren Menge (Konzentration in dem Volumeninkrement) zugeordnet und schließlich gedruckt werden. Geeignete Druckvorrichtungen für das vorliegende Verfahren sind z.B. in US 2017/03,68755 A1 und US 6,070,107 beschrieben.

Bei einer besonders bevorzugten Ausführungsform umfasst das erfindungsgemäße Verfahren außerdem das Aufbringen mindestens einer farbigen Substanz mittels 2D- und/oder 3D-Druck, bevorzugt ebenfalls durch Druck entsprechender, vorzugsweise kleinvolumiger Voxels gemäß dem vorliegenden Verfahren, und/oder durch ein anderes Verfahren, wie bspw. durch zweidimensionales Drucken wie bei einem Tintenstrahldruck, auf dem Objekt oder auf mindestens einem Abschnitt des Objekts derart, dass die aufgebrachte Substanz mindestens eine auf dem Objekt sichtbare Informationsstruktur ausbildet. Dabei kann/können die farbige(n) Substanz(en) getrennt von dem oder den wirkstoffhaltigen Volumeninkrementen aufgebracht werden. Bevorzugt ist es, die Farbsubstanz/en) zusammen mit Volumeninkrementen, die pharmazeutische Wirkstoffe, aufzubringen. In einer Ausführungsform kann so jeweils eine Substanz den oder diejenigen Bereiche bzw. Abschnitte markieren, auf welche(n) der jeweilige Wirkstoff aufgetragen wurde. Diese Ausführungsform kann daher durch Farbcodierung die Information über die in dem Objekt befindlichen Wirkstoffe und deren Verteilung in dem vollständigen Objekt transportieren. In einer Weiterbildung dieser Ausführungsform der Erfindung können in den wirkstoffhaltigen Abschnitten unterschiedliche Mengen bzw. Konzentrationen des jeweiligen Wirkstoffs abgelegt werden, welche wiederum durch die Konzentration der betreffenden Farbsubstanz widergespiegelt werden. Selbstverständlich können auch unterschiedliche Farbsubstanzen z.B. in einem Voxel gemäß der Erfindung Ausführungsform) gemischt werden, so dass durch entsprechende Wahl der Mischung(en) generell das gesamte sichtbare Spektrum verwendet werden kann.

Erfindungsgemäß fallen unter den Begriff "Farbsubstanz" auch solche Substanzen, die lumineszieren, insbesondere fluoreszieren.

Die durch die Farbsubstanz(en) hervorgerufene Informationsstruktur kann vielfältige Informationen abbilden, wobei auch mehrere unterschiedliche Informationsstrukturen durch unterschiedliche Farbsubstanzen zum Einsatz kommen können, die im Wesentlichen frei wählbar und kombinierbar sind. Insbesondere ist es erfindungsgemäß vorgesehen, das die mindestens eine Informationsstruktur Informationen über die Art des oder der in dem Objekt gedruckten Wirkstoffe und/oder über die in dem Objekten vorhandenen Wirkstoffmenge(n) und/oder über den für eine Darreichungsform vorgesehenen Einnahmezeitpunkt oder Einnahmezeitraum und/oder über das für die Darreichungsform vorgesehene Einnahmedatum und/oder über patientenbezogene Daten (wie bspw. Name, Alter, Geschlecht, Medikation und Erkrankung(en) des Patienten) und/oder über den Kostenträger und/oder über den behandelnden Arzt und/oder über das die Darreichungsform bereitstellende pharmazeutische Unternehmen und/oder über die das Objekt, z.B. eine Darreichungsform oder ein Medizinprodukt ausgebende medizinische Einrichtung codiert.

Die Informationsstruktur kann aus einer breiten Palette von Anwendungsmöglichkeiten ausgewählt werden. So kann die Substanz bzw. können die Substanzen in Form von QR-Codes, Buchstaben und/oder Zahlen gedruckt werden. Es können selbstverständlich auch verschiedenste Muster wie bspw. Linien, Gitter, Punkte, flächige Muster usw. gedruckt werden, wobei die bevorzugte Ausführungsform des Voxel-Drucks grundsätzlich die vielfältigsten Möglichkeiten bietet. Das Druckbild des Codes kann somit den Wirkstoff enthalten und codiert gleichzeitig die gewünschten, wie vorstehend beschriebenen Daten über Patient, Arzt, Apotheker und/oder medizinisches oder pharmazeutisches Fachpersonal.

Es ist dem Fachmann ersichtlich, dass in Abhängigkeit von dem gewählten spezifischen additiven Herstellungsverfahren die ggf. vorhandene(n) Farbsubstanz(en) zusammen mit dem oder den pharmazeutischen Wirkstoff(en) in der jeweils gedruckten Basiszusammensetzung vorliegen können. Beim vorliegenden Verfahren ist es bevorzugt, dass die Farbsubstanz (oder mehrere davon) vorzugsweise zusammen mit dem oder den Wirkstoff(en) in der Aufbausubstanz, das/die für ein gegebenes Volumeninkrement vorgesehen ist, vorhanden ist.

Wie bereits vorstehend dargelegt, kann die Darreichungsform verschiedenste Informationsstrukturen enthalten, vorzugsweise solche, die im oben beschriebenen Verfahren genannt sind.

Mit dem Verfahren druckbare wirkstoffhaltige Objekte sind insbesondere halbfeste oder feste pharmazeutische Darreichungsformen, wie z.B. Tabletten, Kapseln, Implantate, Pflaster, Zäpfchen oder Dünnfilme. Mit Hilfe des erfindungsgemäßen Verfahrens herstellbare Tabletten sind vielfältig und schließen Oblongtabletten, Lutschtabletten, Implantattabletten, Multiple-Applikation-Tabletten, Disperstabletten, Retardtabletten, Vaginaltabletten und - zäpfchen, Augentabletten, Manteltabletten, Matrixtabletten, Kautabletten, Filmtabletten, Modified-Release-Tabletten, Lacktabletten und margensaftresistente Tabletten und Drug-Abuse-Deterrent-Tabletten ein.

Weitere, besonders geeignete Objekte sind Medizinprodukte wie z.B. wirkstoffhaltige topische Arzneiformen, Kontaktlinsen, Pflaster, welche vorzugsweise den oder die Wirkstoffe zur lokalen Anwendung freisetzen.

Die vorliegende Erfindung bezieht sich auch auf die durch das Verfahren hergestellten zwei- oder dreidimensionale Objekte (vorzugsweise die vorstehend genannten), wobei diese mindestens einen pharmazeutischen Wirkstoff enthalten.

Die vorliegende Erfindung wird durch die beigefügten Figuren näher erläutert.

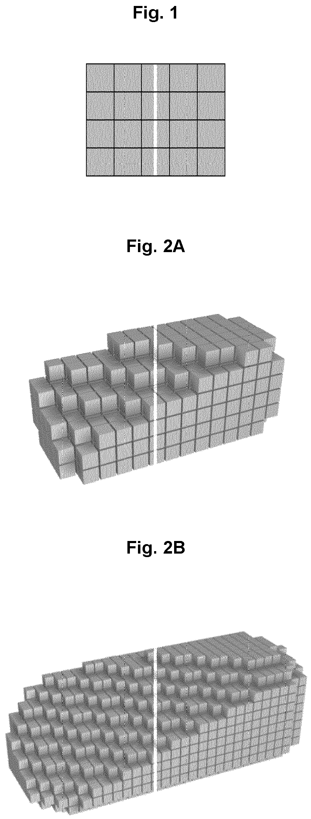

Fig. 1 zeigt eine schematische Darstellung eines Querschnitts eines dreidimensionalen Objekts, dass in einzelne Volumeninkremente (im Querschnitt quadratisch) unterteilt ist (nachfolgend auch "voxelisiert" genannt).
Fig. 2 zeigt schematische Darstellungen eines in würfelförmige Volumeninkremente unterteiltes (also voxelisiertes) dreidimensionalen Objekts, wobei in Fig. 2A die Auflösung des Objekts durch größere Voxel kleiner ist (also gröbere Auflösung) und in Fig. 2B durch Unterteilung in kleinere Volumeninkremente die Auflösung größer ist (also feinere Auflösung).

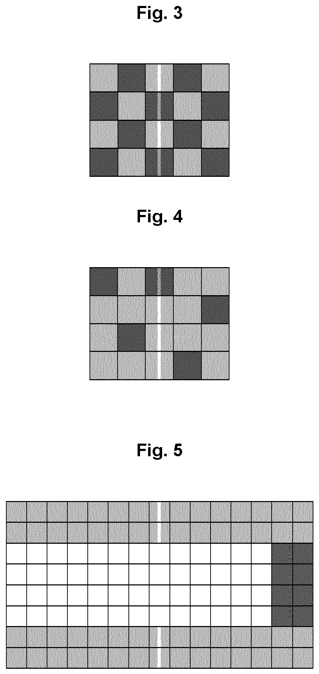

Fig. 3 zeigt eine schematische Darstellung eines Querschnitts eines dreidimensionalen Objekts, bei dem alternierend Volumeninkremente (in dem Querschnitt quadratisch dargestellt) ohne Wirkstoff bzw. einen wirkstoffenthaltenden Voxel (hell dargestellte Voxel) und Volumeninkrementen mit Wirkstoff (dunkel dargestellte Voxel) vorhanden sind, wobei sich eine Konzentration der Voxel mit Wirkstoff von z.B. 50 % ergibt.
Fig. 4 zeigt eine schematische Darstellung eines Querschnitts eines dreidimensionalen Objekts, bei dem einzelne Voxel Wirkstoff enthalten (dunkel dargestellt), so dass 25 % der Voxel Wirkstoff enthalten. Der Anteil an wirkstoffenthaltenden Voxel zu wirkstofffreien Voxel bzw. der Volumenanteil den verschiedenen Wirkstoffen enthaltenden Voxeln kann erfindungsgemäß beliebig zwischen 0,1 und 99,9 % variiert werden.
Fig. 5 zeigt eine schematische Darstellung eines Querschnitts eines dreidimensionalen Objekts, bei dem wirkstoffhaltige Voxel (dunkel) im inneren zusammengefasst sind und von nicht wirkstoffhaltigen Voxeln (hell) umgeben sind, so dass diese die wirkstoffhaltigen Voxel von der Umgebung wie z.B. einer Körperflüssigkeit schützen können. Durch einen derartigen Aufbau kann die Freisetzung des Wirkstoffs gesteuert werden. In anderen Ausführungsformen kann die Freigabesteuerung, wie bereits vorstehend offenbart, mittels pH-abhängig löslicher bzw. abbaubarer Polymere erreicht werden. Die verschiedenen Konzentrationen und Materialien können durch die vorliegend offenbarte und beschriebene Vorgehensweise sehr hochauflösende Arzneiformen ergeben. So können in Fig. 5. z.B. die dunklen Voxel jeweils Fig. 3 entsprechen und die hellen Voxel der Fig. 4. Auf diese Weise sind auch bspw. Tabletten mit Abuse-Deterrent-Eigenschaften, wie bereits vorstehend dargelegt, herstellbar.

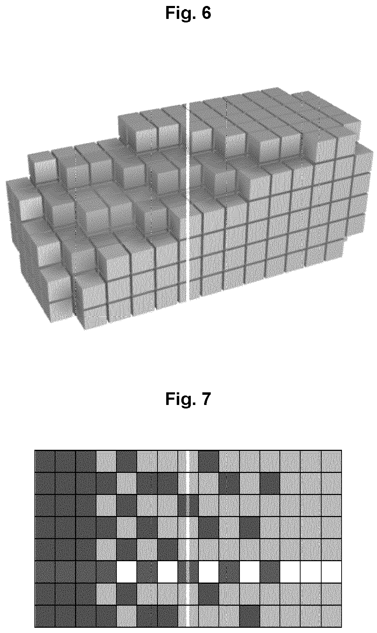

Fig. 6 zeigt eine schematische Darstellung eines erfindungsgemäß herstellbaren Objekts, welche die in Fig. 5 erläuterte Ausführungsform weiterbildet: Voxel mit einem ersten Wirkstoff (blau) sind von Voxeln mit einem zweiten Wirkstoff (rot) umgeben, die wiederum von wirkstofffreien Voxeln umgeben sind. Diese Ausführungsform kann z.B. auch derart ausgestaltet werden, dass nicht unterschiedliche Wirkstoffe verwendet werden, sondern unterschiedliche Wirkstoffkonzentrationen.
Fig. 7 zeigt eine schematische Darstellung eines Querschnitts eines dreidimensionalen Objekts, bei dem durch von links nach rechts abnehmende Dichte der wirkstoffhaltigen Voxel (dunkel) ein Konzentrationsgefälle des Wirkstoffs in dem Objekt aufgebaut wird.

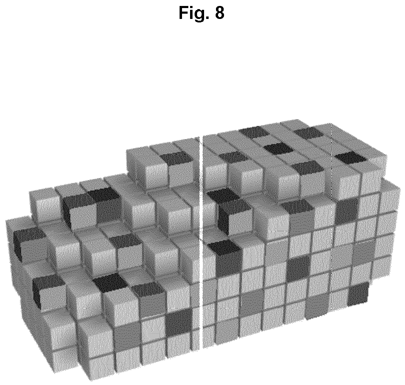

Fig. 8 zeigt eine schematische Darstellung eines erfindungsgemäß herstellbaren Objekts, bei dem Voxel mit drei unterschiedlichen Wirkstoffen oder Wirkstoffkonzentrationen (blau, rot, grün) in dem Objekt verteilt und von nicht wirkstoffhaltigen Voxels umgeben sind.

In beispielhaften Ausführungsformen kann durch das erfindungsgemäße Verfahren z.B. die Wirkstofffreigabe bei Patienten mit Herzerkrankungen oder Bluthochdruck den physiologischen Bedürfnissen angepasst werden. So können z.B. ein Cholesterinsenker (bspw. ein Statin) und zwei blutdrucksenkende Substanzen (z.B. Sartan und Betablocker) derart in eine Arzneiform verdruckt werden, dass die Wirkstofffreisetzung über 24 Stunden den physiologischen Bedingungen des Patienten optimal entspricht. Bei einer morgentlichen Einnahme der Arzneiform durch den Patienten können die blutdrucksenkenden Substanzen schnell freigesetzt werden, während der Cholesterinsenker erst mit 12-16 Stunden Verzögerung freigesetzt wird, um die nächtliche Cholesterinsynthese zu reduzieren.

In weiteren Beispielen des erfindungsgemäßen Verfahrens kann eine Auftitration neu diagnostizierter Patienten mit Erkrankungen wie Parkinson, Multiple Sklerose, Herzinfarkt, Schlaganfällen oder transplatierte Patienten und Anwendungen in der Geriatrie bzw. Pädiatrie bereitgestellt werden. In all diesen Fällen kann die patientenspezifische Dosierung oder Freigabe von Wirkstoffen die Wirkung optimiert und die Nebenwirkungen reduziert werden.

## Patentansprüche

1. Verfahren zur Herstellung eines Objekts, das mindestens einen pharmazeutischen Wirkstoff enthält, umfassend die Schritte:
(i) Erstellen einer zwei- oder dreidimensionalen Widergabe des herzustellenden Objekts durch vordefinierte Volumeninkremente;
(ii) Drucken eines vordefinierten Volumeninkrements auf eine Aufbauvorrichtung oder auf ein auf der Aufbauvorrichtung angeordnetes Objekt;
(iii) Drucken eines weiteren Volumeninkrements derart, dass sich die Volumeninkremente mindestens teilweise berühren oder überlappen; und
(iv) Wiederholen der Schritte (ii) und (iii), bis das Objekt erstellt ist;
wobei mindestens eines der Volumeninkremente mindestens einen pharmazeutischen Wirkstoff enthält und die Volumeninkremente aus einer bei einer für den mindestens einen Wirkstoff verträglichen Drucktemperatur fließfähigen Basiszusammensetzung oder Basissubstanz umfassen, die sich nach dem Drucken des jeweiligen Volumeninkrements verfestigt und/oder verklebt werden,
**dadurch gekennzeichnet, dass**
das Volumen der Volumeninkremente unterschiedlich ist derart, dass das Volumen der Volumeninkremente in dem gedruckten Objekt von außen nach innen ansteigt.

2. Verfahren nach Anspruch 1, wobei die Schritte (ii) und (iii) derart erfolgen, dass die Volumeninkremente schichtweise gedruckt werden.

3. Verfahren nach Anspruch 1 oder 2, wobei jedes Volumeninkrement einen Wirkstoff enthält.

4. Verfahren nach Anspruch 1 oder 2, wobei unterschiedliche Volumeninkremente unterschiedliche Wirkstoffe und/oder unterschiedliche Wirkstoffmengen und/oder unterschiedliche Basiszusammensetzungen bzw. Basissubstanzen aufweisen.

5. Verfahren nach einem der vorhergehenden Ansprüche, wobei die Form der Volumeninkremente aus der Gruppe, bestehend aus Tropfen, Kugeln, Punkten, Zylindern, Würfeln und Quadern, ausgewählt ist.

6. Verfahren nach einem der vorhergehenden Ansprüche, wobei wirkstoffhaltige Volumeninkremente derart aufgebaut sind und gedruckt werden, dass mindestens eine erste Gruppe von sich berührenden oder überlappenden wirkstoffhaltigen Volumeninkrementen eine gleiche Wirkstoffmenge enthält und mindestens eine zweite Gruppe von sich berührenden Volumeninkrementen eine Wirkstoffmenge enthält, die von der Wirkstoffmenge der ersten Gruppe verschieden ist.

7. Verfahren nach einem der vorhergehenden Ansprüche, wobei mindestens eine erste Gruppe wirkstoffhaltiger Volumeninkremente vorhanden ist, die einen ersten pharmazeutischen Wirkstoff enthält und mindestens eine zweite Gruppe wirkstoffhaltiger Volumeninkremente vorhanden ist, die einen vom ersten Wirkstoff verschiedenen zweiten pharmazeutischen Wirkstoff enthält.

8. Verfahren nach einem der vorhergehenden Ansprüche, wobei die Volumeninkremente derart gedruckt werden, dass wirkstoffhaltige Volumeninkremente innerhalb des Objekts eine oder mehrere Gruppen bilden, die von nicht-wirkstoffhaltigen Volumeninkrementen umgeben sind, welche die wirkstoffhaltigen Volumeninkremente von der Umgebung abschirmen.

9. Verfahren nach Anspruch 8, wobei die wirkstoffhaltigen Volumeninkremente in dem Objekt einen wirkstoffhaltigen Kern bilden, um den herum nicht-wirkstoffhaltige Volumeninkremente angeordnet sind.

10. Verfahren nach Anspruch 9, wobei die Volumeninkremente derart gedruckt werden, dass wirkstoffhaltige Volumeninkremente mehrere Gruppen bilden, die jeweils von nicht-wirkstoffhaltigen Volumeninkrementen umgeben sind.

11. Verfahren nach Anspruch 9 oder 10, wobei die nicht-wirkstoffhaltigen Volumeninkrementen eine Substanz enthalten, welche die Wirkung des oder der Wirkstoffe, vorzugsweise eines oder mehre Wirkstoffe mit Suchtpotential, in den wirkstoffhaltigen Volumeninkrementen mindestens begrenzt, wenn das gedruckte Objekt zerkleinert wird.

12. Verfahren nach Anspruch 9, wobei die nicht-wirkstoffhaltigen Volumeninkremente derart gedruckt werden, dass Sie eine oder mehrere pH-abhängig im Darm eines Patienten abbaubare Schicht(en) um einen wirkstoffhaltigen Kern bilden.

13. Verfahren nach Anspruch 10 oder 11, wobei nicht-wirkstoffhaltige Volumeninkremente eine Retard-Freisetzung des Wirkstoffs oder der Wirkstoffe bewirkten.

14. Verfahren nach einem der vorhergehenden Ansprüche, wobei das wirkstoffhaltige Objekt eine halbfeste oder feste pharmazeutische Darreichungsform, vorzugsweise ausgewählt aus der Gruppe, bestehend aus Tabletten, Kapseln, Zäpfchen, Pflaster oder Dünnfilmen, oder ein Medizinprodukt ist.

15. Dreidimensionales Objekt, das mindestens einen pharmazeutischen Wirkstoff enthält, hergestellt durch das Verfahren nach einem der vorhergehenden Ansprüche.

## Claims

1. A method for producing an object containing at least one pharmaceutical active agent, comprising the steps:
(i) generating a two- or three-dimensional representation of the object to be manufactured using predefined volume increments;
(ii) printing a predefined volume increment onto a building platfoum or onto an object placed on the building platform;
(iii) Printing a further volume increment in such a way that the volume increments at least partially contact or overlap each other; and
(iv) repeating steps (ii) and (iii) until the object is generated;
wherein at least one of the volume increments contains at least one pharmaceutical active agent and the volume increments comprise a base composition or base substance being flowable at a printing temperature compatible with the at least one active agent and that solidifies and/or bonds after printing of the respective volume increment,
**characterized in that**
the volume of the volume increments is different in a manner such that the volume of the volume increments in the printed object increases from the outside to the inside.

2. The method of claim 1, wherein steps (ii) and (iii) are carried out in a manner such that the volume increments are printed in layers.

3. The method of claim 1 or 2, wherein each volume increment contains an active agent.

4. The method of claim 1 or 2, wherein different volume increments have different active agents and/or different amounts of active agents and/or different base compositions or base substances.

5. The method according to any one of the preceding claims, wherein the shape of the volume increments is selected from the group consisting of drops, spheres, dots, cylinders, cubes, and cuboids.

6. The method according to any one of the preceding claims, wherein active agent-containing volume increments are constructed and printed in such a way that at least a first group of contacting or overlapping active agent-containing volume increments contains an equal amount of active agent and at least a second group of contacting volume increments contains an amount of active agent that differs from the amount of active agent in the first group.

7. The method according to any one of the preceding claims, wherein at least one first group of active agent-containing volume increments is present, which contains a first pharmaceutical active agent, and at least one second group of active agent-containing volume increments is present, which contains a second pharmaceutical active agent that is different from the first active agent.

8. The method according to any one of the preceding claims, wherein the volume increments are printed in a manner such that volume increments containing active agent form one or more groups within the object, which are surrounded by volume increments not containing active agents, which shield the volume increments containing active agents from the environment.

9. The method of claim 8, wherein the volume increments containing active agent form an active agent-containing core in the object, around which volume increments not containing active agent are arranged.

10. The method of claim 9, wherein the volume increments are printed in such a manner such that active agent-containing volume increments form several groups, each of which is surrounded by non-active agent-containing volume increments.

11. The method of claim 9 or 10, wherein the non-active agent-containing volume increments contain a substance which at least limits the effect of the active agent or agents, preferably one or more active agents with addictive potential, in the active agent-containing volume increments when the printed object is disintegrated.

12. The method of claim 9, wherein the non-active agent-containing volume increments are printed in a manner such that they form one or more layers around an active agent-containing core, which layer(s) is/are pH dependently degraded in the intestine of a patient.

13. The method of claim 10 or 11, wherein non-active agent-containing volume increments cause a delayed release of the active agent or active agents.

14. The method according to any one of the preceding claims, wherein the active agent-containing object is a semi-solid or solid pharmaceutical dosage form, preferably selected from the group consisting of tablets, capsules, suppositories, patches, or thin films, or medical device.

15. Three-dimensional object containing at least one pharmaceutical active agent, produced by the method according to any one of the preceding claims.

## Revendications

1. Procédé de fabrication d'un objet contenant au moins un principe actif pharmaceutique, comprenant les étapes suivantes :
(i) création d'une représentation en deux ou trois dimensions de l'objet à fabriquer par incréments de volume prédéfinis ;
(ii) imprimer un incrément de volume prédéfini sur un dispositif de construction ou sur un objet placé sur le dispositif de construction ;
(iii) imprimer un autre incrément de volume de telle sorte que les incréments de volume se touchent ou se chevauchent au moins partiellement ; et
(iv) répéter les étapes (ii) et (iii) jusqu'à ce que l'objet soit créé ;
dans lequel au moins l'un des incréments de volume contient au moins un ingrédient pharmaceutique actif et les incréments de volume comprennent une composition de base ou une substance de base qui est fluide à une température d'impression compatible avec au moins un ingrédient actif et qui se solidifie et/ou se lie après l'impression de l'incrément de volume respectif,
**caractérisé en ce que**
le volume des incréments de volume est différent de telle sorte que le volume des incréments de volume dans l'objet imprimé augmente de l'extérieur vers l'intérieur.

2. Procédé selon la revendication 1, dans lequel les étapes (ii) et (iii) sont réalisées de telle sorte que les incréments de volume sont imprimés en couches.

3. Procédé selon la revendication 1 ou 2, dans lequel chaque incrément de volume contient un ingrédient actif.

4. Procédé selon la revendication 1 ou 2, dans lequel différents incréments de volume ont différents ingrédients actifs et/ou différentes quantités d'ingrédients actifs et/ou différentes compositions de base ou substances de base.

5. Procédé selon l'une des revendications précédentes, dans lequel la forme des incréments de volume est choisie parmi le groupe comprenant des gouttes, des sphères, des points, des cylindres, des cubes et des prismes rectangulaires.

6. Procédé selon l'une des revendications précédentes, dans lequel les incréments de volume contenant des ingrédients actifs sont construits et imprimés de telle manière qu'au moins un premier groupe d'incréments de volume contenant des ingrédients actifs en contact ou se chevauchant contient une quantité égale d'ingrédient actif et qu'au moins un deuxième groupe d'incréments de volume en contact contient une quantité d'ingrédient actif qui diffère de la quantité d'ingrédient actif dans le premier groupe.

7. Procédé selon l'une des revendications précédentes, dans lequel au moins un premier groupe d'incréments de volume contenant un principe actif est présent, qui contient un premier principe actif pharmaceutique, et au moins un deuxième groupe d'incréments de volume contenant un principe actif est présent, qui contient un deuxième principe actif pharmaceutique différent du premier principe actif.

8. Procédé selon l'une des revendications précédentes, dans lequel les incréments de volume sont imprimés de telle manière que les incréments de volume contenant des ingrédients actifs forment un ou plusieurs groupes à l'intérieur de l'objet, qui sont entourés par des incréments de volume ne contenant pas d'ingrédients actifs, qui protègent les incréments de volume contenant des ingrédients actifs de l'environnement.

9. Procédé selon la revendication 8, dans lequel les incréments de volume contenant des ingrédients actifs forment un noyau contenant des ingrédients actifs dans l'objet, autour duquel sont disposés des incréments de volume ne contenant pas d'ingrédients actifs.

10. Procédé selon la revendication 9, dans lequel les incréments de volume sont imprimés de telle manière qu' , les incréments de volume contenant des ingrédients actifs forment plusieurs groupes, chacun étant entouré d'incréments de volume ne contenant pas d'ingrédients actifs.

11. Procédé selon la revendication 9 ou 10, dans lequel les incréments de volume ne contenant pas d'ingrédient actif contiennent une substance qui limite au moins l'effet du ou des ingrédients actifs, de préférence un ou plusieurs ingrédients actifs présentant un potentiel addictif, dans les incréments de volume contenant l'ingrédient actif lorsque l'objet imprimé est écrasé.

12. Procédé selon la revendication 9, dans lequel les incréments de volume ne contenant pas d'ingrédient actif sont imprimés de telle manière qu'ils forment une ou plusieurs couches dépendantes du pH dégradables dans l'intestin d'un patient autour d'un noyau contenant un ingrédient actif.

13. Procédé selon la revendication 10 ou 11, dans lequel les incréments de volume ne contenant pas de principe actif provoquent une libération retardée du ou des principes actifs.

14. Procédé selon l'une des revendications précédentes, dans lequel l'objet contenant le principe actif est une forme posologique pharmaceutique semi-solide ou solide, de préférence choisie dans le groupe constitué par les comprimés, les gélules, les suppositoires, les patchs ou les films minces, ou un dispositif médical.

15. Objet tridimensionnel contenant au moins un principe actif pharmaceutique, produit selon le procédé selon l'une des revendications précédentes.
